(19) Europäisches Patentamt
European Patent Office
Office européen des brevets

(11) Publication number: 0 008 139
B1

(12) # EUROPEAN PATENT SPECIFICATION

(45) Date of publication of patent specification: 24.02.82

(51) Int. Cl.³: C 07 C 11/12, C 07 C 2/40, B 01 J 31/24

(21) Application number: 79200392.3

(22) Date of filing: 13.07.79

(54) A process for preparing 1,7-octadiene.

(30) Priority: 24.07.78 US 927693

(43) Date of publication of application: 20.02.80 Bulletin 80/4

(45) Publication of the grant of the patent: 24.02.82 Bulletin 82/8

(84) Designated Contracting States: DE FR GB NL

(56) References cited:
GB - A - 1 341 324
US - A - 3 732 328
US - A - 3 823 199

CHEMICAL ABSTRACTS, Vol. 85, No. 7
16 August 1976
Columbus, Ohio, USA
P. ROFFIA et al. "1,7-Octadiene"
page 473

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Nozaki, Kenzie
1407, Lakecliff Drive
Houston, Texas 77077 (US)

(74) Representative: Puister, A.T., Mr. et al.
P.O. Box 302
NL-2501 CH The Hague (NL)

EP 0 008 139 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# A process for preparing 1,7-octadiene

The invention relates to a process for preparing 1,7-octadiene by hydrodimerizing 1,3-butadiene in the presence of a palladium catalyst, a tertiary phosphine, formic acid and a solvent which is a dialkyl sulphoxide and/or a dialkylformamide and in the absence of a base.

Linear dimerization of butadiene (1,3-butadiene) provides a source of $C_8$-unsaturated hydrocarbon intermediates useful for the synthesis of diacids, diesters, diols or diamines. A particularly preferred dimer is 1,7-octadiene which has terminal double bonds and allows the production of product having only terminal functional groups.

U.S. patent specification No. 3,732,328 discloses a process of the type described in the beginning. Trialkyl-, mixed alkylaryl- or triarylphosphines are mentioned as useful, specifically noting triphenylphosphine. The yield of 1,7-octadiene is much lower than that of the present invention.

British patent specification No. 1,341,324 discloses the preparation of mixtures of octadienes by reacting butadiene in the presence of a palladium compound, formic acid, an amine solvent and a tertiary phosphine. Trialkyl-, mixed alkylaryl- or triarylphosphines are mentioned as useful, specifically noting triphenylphosphine. The yield of 1,7-octadiene is very low.

Tetrahedron Letters No. 2, pp. 163—164 discloses the production of mixtures of octadienes by reacting butadiene in the presence of palladium acetate, triphenylphosphine, formic acid and dimethylformamide. The yield of 1,7-octadiene is much lower than that of the present invention.

U.S. patent specification No. 3,823,199 discloses the preparation of mixtures of octadienes by reacting butadiene in the presence of a palladium compound, formic acid, a non-polar solvent and a tertiary phosphine. Trialkylphosphines are mentioned as useful, specifically noting triethyl- and tributylphosphine. The applicants have found that carrying out the process described in the beginning using triethyl- or tributylphosphine gives a very low yield of 1,7-octadiene.

It has now been found that the use of members of a specific group of tertiary phosphines not only allows a very high selectivity to but also a very high yield of 1,7-octadiene.

Accordingly, the invention provides a process for preparing 1,7-octadiene by hydrodimerizing butadiene in the presence of a palladium catalyst, a tertiary phosphine, formic acid and a solvent which is a dialkyl sulphoxide and/or a dialkylformamide (by "dialkyl" it is meant that the sulphur and nitrogen atoms in the latter two groups of compounds are connected to two different carbon atoms) and in the absence of a base, characterized in that the tertiary phosphine is a phosphine of formula:

$$R_1R_2R_3P$$

wherein $R_1$ is a branched alkyl group having from 3 to 10 carbon atoms with branching occurring at a carbon atom no more than two carbon atoms from the phosphorus atom and $R_2$ and $R_3$ are $R_1$ or independently are alkyl, alkenyl, aralkyl, cycloalkyl or aryl having from 1 to 10 carbon atoms.

European patent publication No. 4409 discloses the production of 1,7-octadiene by reacting butadiene in the presence of a palladium catalyst, triisopropylphosphine, formic acid, a base and optionally a solvent. The present invention in contradistinction operates without the presence of a base.

If the palladium compound added is a palladium tertiary phosphine complex then it is not necessary to add a separate tertiary phosphine. The use of the tertiary phosphine in combination with the solvents of this invention allows the hydrodimerization reaction to be carried out in high yield without a base being required.

The catalyst used in the process of this invention is palladium or a palladium compound which may be complexed with a tris-organophosphine. The palladium may be in any of its possible valence states, e.g., 0, +2, etc. Suitable palladium compounds include the palladium carboxylates, particularly palladium carboxylates derived from alkanoic acids containing up to six carbon atoms per molecule, such as palladium acetate, complexes, such as palladium acetylacetonate, bis-benzonitrile palladium(II) and lithium palladous chloride as well as the palladium halides, nitrates and sulphates, such as palladous chloride, palladium nitrate ($Pd(NO_3)_2(OH)_2$) and palladium sulphate. Preferred catalysts are palladium acetylacetonate or palladium acetate. Suitable reduced palladium-phosphine complexes are $Pd(R_3P)_2$ or $Pd(R_3P)_3$. A preferred complex is Pd(0)-triisopropylphosphine. The palladium is present in the reaction mixture in catalytic amounts; preferably from about 1 to about $10^{-6}$ molar and more preferably from about $10^{-1}$ to about $10^{-4}$ molar.

The palladium compounds complexed with a tris-organophosphine are typically prepared by reacting the tertiary phosphine with the appropriate palladium compound as, for example, represented by the following equations:

$$2R_3P + (PhCN)_2PdCl_2 \rightarrow (R_3P)_2PdCl_2$$

$$(R_3P)_3PdCl_3 + Ag_2CO_3 \rightarrow (R_3P)_2PdCO_3$$

$$(R_3P)_2PdO_2 + SO_2 \rightarrow (R_3P)_2PdSO_4$$

$$(R_3P)_2PdO_2 + N_2O_4 \rightarrow (R_3P)_2Pd(NO_3)_2$$

where $R_3P$ is the tris-organophosphine of the invention, or may be made in situ by adding the palladium compound and the phosphine directly to the reactor.

The catalyst may be pretreated to enhance reactivity by contacting it with a reducing agent at a temperature of from about 20 to about 90°C for from about 0.1 to 5 hours. The reducing agent may be gaseous, solid or liquid. Examples of gaseous agents are hydrogen and carbon monoxide. Examples of liquid or solid reducing agents are hydrazine, $NaBH_4$, $NaOCH_3$, $(isopropyl)_3P$, Cu, Na and alkyls, etc. The reduction may be carried out in a separate autoclave or preferably is carried out in the hydrodimerization reactor prior to the introduction of the butadiene. The palladium compound tri-organophosphine complex may be dissolved in the solvent used in this invention prior to reduction.

The tris-organophosphine utilized in the process of this invention have the following general formula:

$$R_1R_2R_3P$$

wherein $R_1$ is a branched alkyl group having from 3 to 10 carbon atoms with branching occurring at a carbon atom no more than two carbon atoms from the phosphorus atom and $R_2$ and $R_3$ are $R_1$ or independently are alkyl, alkenyl, aralkyl, cycloalkyl or aryl groups having from 1 to 10 carbon atoms.

By branched is meant that the $\alpha$- and $\beta$-carbon atoms, relative to the phosphorus atom, of the stated groups may not both be $-CH_2-$linkages.

Illustrative of the $R_1$ moiety are isopropyl, sec.-butyl, tert.-butyl, isobutyl, neopentyl, sec.-pentyl, tert.-pentyl, 2-methylbutyl, sec.-hexyl, tert.-hexyl and 2,2-dimethylpropyl.

Illustrative of $R_2$ and $R_3$ are, for example, methyl, ethyl, propyl, butyl, pentyl, hexyl, actyl, nonyl and decyl for alkyl; allyl, crotyl and methallyl for alkenyl and phenyl, tolyl, xylyl, ethylphenyl, propylphenyl for aryl. Two or more of the instant phosphines may be used in the same reaction or another phosphine may be replaced by reacting in situ one of the instant phosphines. Preferred phosphines are triisopropylphosphine, tri-tert.-butylphosphine, tri-sec.-butylphosphine, di-tert.-butyl-normal-butylphosphine, tri-isobutylphosphine and tert.-butyl-dibenzylphosphine and mixtures thereof.

The molar ratio of tris-organophosphine to palladium is at least 1. Preferably, the molar ratio of phosphine to palladium ranges from about 1:1 to about 20:1 and more preferably from about 2:1 to about 5:1.

The solvent needed to provide the high yields of this invention is a dialkyl sulphoxide and/or a dialkylformamide. The alkyl moieties have from 1 to 6 carbon atoms. Included within the above definition of dialkyl would be polyalkylene, e.g., an alkylene group doubly attached to the sulphinyl or carbonylamino group as, for example, tetrahydrothiophene 1-oxide or N-methylpyrrolidinone. Preferred solvents are dimethyl sulphoxide and dimethylformamide.

The addition of carbon dioxide to the reaction system has been found to increase the extent of butadiene conversion, but does not affect the selectivity. When it is desired to use carbon dioxide to increase the conversion rate, the partial pressure of the $CO_2$ in the reaction system may be from about 0.5 to about 70 bars. Since the carbon dioxide is a by-product of the process, it is possible to generate sufficient carbon dioxide *in situ* to enhance the conversion rates.

The process can be either continuous or batch. The reaction temperature of the process is not critical, however, it is preferred to maintain the reaction between about 0 to about 100°C, preferably between about 20 to about 70°C. The process is conducted at the reaction temperature. Typically the pressure is autogenous.

The process of this invention is particularly useful when applied to a butadiene/isobutane/n-butenes (BBB) stream from an oil pyrolysis unit. These BBB streams are the $C_4$ cut from a thermal cracking unit typically containing 30—40% butadiene, 20—35% isobutene and 20—30% n-butenes and many other minor components.

The process of this invention will now be illustrated by reference to the following Examples. The Examples and Comparative Experiments are indicated with figures and capitals, respectively.

### Examples 1—12 and Comparative Experiments A—Q

To an 80 ml glass-lined autoclave were charged the palladium compound (0.027 mmol) listed in column 2 of Table I triisopropylphosphine (0.054 mmol), formic acid (18.5 mmol), butadiene (2 g) and the solvent (10 ml) listed in column 3 of Table I. The stirred reactor was heated to 60°C for 1 hour. The reactor was then cooled and the product was analyzed by gas chromatography. The results are shown in column 4 of Table I.

TABLE I

| Example or Comparative Experiment | Pd compound | Solvent | Butadiene conversion to 1,7-octadiene, % |
|---|---|---|---|
| 1 | $Pd(AcAc)_2$[a] | Dimethyl sulphoxide | 77 |
| 2 | $Pd(AcAc)_2$ | Tetrahydrothiophene 1-oxide | 83 |
| 3 | $Pd(AcAc)_2$ | Dimethylformamide | 98 |
| 4 | $Pd(AcAc)_2$ | N-methylpyrrolidinone | 75 |
| A | $Pd(AcAc)_2$ | Dimethylacetamide | 70 |
| B | $Pd(AcAc)_2$ | Pyridine | 30 |
| C | $Pd(AcAc)_2$ | Tetrahydrofuran | 24 |
| D | $Pd(AcAc)_2$ | Quinoline | 17 |
| E | $Pd(AcAc)_2$ | Tetrahydrothiophene 1,1-dioxide | 3 |
| F | $Pd(AcAc)_2$ | Nitromethane | 0 |
| G | $Pd(AcAc)_2$ | Ethyl sulphide | 0 |
| H | $Pd(AcAc)_2$ | None | 0 |
| 5 | $Pd(OAc)_2$[b] | Dimethyl sulphoxide | 78 |
| 6 | $Pd(OAc)_2$ | Dimethylformamide | 66 |
| 7 | $Pd(OAc)_2$ | N-methylpyrrolidinone | 40 |
| I | $Pd(OAc)_2$ | 2,6-lutidine | 35 |
| J | $Pd(OAc)_2$ | Dimethylacetamide | 4.5 |
| K | $Pd(OAc)_2$ | Acetamide | 0.8 |
| L | $Pd(OAc)_2$ | Formamide | 0 |
| M | $Pd(OAc)_2$ | Acetonitrile | 0 |
| N | $Pd(OAc)_2$ | Dimethylaniline | 0 |
| 8 | $PdSO_4$ | Dimethyl sulphoxide | 68 |
| 9 | $PdSO_4$ | Dimethylformamide | 98 |
| O | $PdSO_4$ | Pyridine | 18 |
| P | $PdSO_4$ | Tetrahydrofuran | 9 |

TABLE I (Continued)

| Example or Comparative Experiment | Pd compound | Solvent | Butadiene conversion to 1,7-octadiene, % |
|---|---|---|---|
| 10 | $Pd(NO_3)_2(OH)_2$ | Dimethyl sulphoxide | 21 |
| 11 | $Pd(NO_3)_2(OH)_2$ | Dimethylformamide | 2 |
| 12 | $PdCl_2$ | Dimethylformamide | 0.9 |
| Q | $Pd(\phi_3P)_4$ [c] | Dimethylformamide | 0.3 |

[a] AcAc = acetylacetonate;
[b] OAc = acetate;
[c] No (Isopropyl)$_3$P added; $\phi_3P$ represents triphenylphosphine.

Examples 13—17 and Comparative Experiments R—T

To an 80 ml glass-lined autoclave were charged the palladium compound (0.025 mmol) listed in column 2 of Table II, triisopropylphosphine (0.054 mmol) formic acid (18.5 mmol), butadiene (2 g) and the solvent (10 ml) listed in column 3 of Table II. The stirred reactor was heated to 40°C for 2 hours. The reaction was then cooled and the product was analyzed by gas chromatography. The results are shown in columns 4 and 5 of Table II. Triisopropylphosphine and triphenylphosphine are abbreviated by i-Pr$_3$P and $\phi_3P$.

TABLE II

| Example or Comparative Experiment | Pd compound | Solvent | Other changes | Conversion to 1,7-octa-diene, % | Conversion to 1,6-octa-diene, % |
|---|---|---|---|---|---|
| 13 | $Pd(AcAc)_2$ | Dimethylformamide | | 55.0 | 1.0 |
| R | $Pd(AcAc)_2$ | Dimethylformamide | No P | 5.8 | 43.0 |
| S | $Pd(AcAc)_2$ | Dimethylformamide | $\phi_3P$ in place of i-Pr$_3$P | 5.8 | 0.9 |
| 14 | $Pd(AcAc)_2$ | Dimethyl sulphoxide | | 81.0 | 2.0 |
| 15 | $Pd(AcAc)_2$ | N-Me-pyrrolidinone | | 40.0 | |
| T | $Pd(AcAc)_2$ | Pyridine | | 0.6 | |
| 16 | $Pd(OAc)_2$ | Dimethylformamide | | 92.0 | 4.0 |
| 17 | $PdSO_4$ | Dimethylformamide | | 37.0 | |

Examples 18—21 and Comparative Experiments U—X

To an 80 ml glass-lined autoclave were charged palladium acetylacetonate (0.027 mmol), the phosphine listed below (0.054 mmol), dimethylsulphoxide (10 ml), formic acid (18.5 mmol) and butadiene (2 g). The stirred reactor was heated to 40°C for 2 hours. The reactor was then cooled and the product was analyzed by gas chromatography. The results are shown in Table III.

TABLE III

| Example or Comparative Experiment | Phosphine | 1,7-octadiene Conversion, % | Selectivity, % |
|---|---|---|---|
| 18 | $(isopropyl)_3P$ | 81 | 97.5 |
| 19 | $(t.\text{-}butyl)_2$-n-butyl-P | 39 | 99 |
| 20 | $(sec.\text{-}butyl)_3P$ | 20 | 98.5 |
| 21 | $(t.\text{-}butyl)_3P$ | 66 | 98.5 |
| U | $(n\text{-}butyl)_3P$ | 9.5 | 86 |
| V | $(n\text{-}propyl)_3P$ | 4.0 | 87 |
| W | $(ethyl)_3P$ | 6 | 85 |
| X | $(phenyl)_3P$ | 5.7 | 86 |

Example 22

A zero-valent palladium complex was prepared according to the teachings of W. Kuran and A. Musco, *Inorganic Chimica Acta,* Vol. 12, 1975, pp. 187—193. Pd(0)-$(triisopropylphosphine)_3$ complex (0.0068 mmol), formic acid (18.5 mmol), dimethylsulphoxide (10 ml) and butadiene (2 g) were charged to an 80 ml glass-lined autoclave. The stirred reactor was heated to 60°C for one hour, cooled and the product analyzed by gas chromatography. Conversion of butadiene to 1,7-octadiene was 80.4% in 97.3% selectivity.

**Claims**

1. A process for preparing 1,7-octadiene by hydrodimerizing 1,3-butadiene in the presence of a palladium catalyst, a tertiary phosphine, formic acid and a solvent which is a dialkyl sulphoxide and/or a dialkylformamide (by "dialkyl" it is meant that the sulphur and nitrogen atoms in the latter two groups of compounds are connected to two different carbon atoms) and in the absence of a base, characterized in that the tertiary phosphine is a phosphine of formula:

$$R_1R_2R_3P$$

wherein $R_1$ is a branched alkyl group having from 3 to 10 carbon atoms with branching occurring at a carbon atom no more than two carbon atoms from the phosphorus atom and $R_2$ and $R_3$ are $R_1$ or independently are alkyl, alkenyl, aralkyl, cycloalkyl or aryl groups having from 1 to 10 carbon atoms.

2. A process as claimed in claim 1, characterized in that the tertiary phosphine is triisopropylphosphine, di-tert.-butyl-n-butylphosphine, tri-secondary-butylphosphine or tri-tertiary-butylphosphine or a mixture of two or more of such phosphines.

3. A process as claimed in claim 1 or 2, characterized in that the palladium catalyst is $Pd(R_1R_2R_3P)_2$ or $Pd(R_1R_2R_3)_3$, wherein $R_1$, $R_2$ and $R_3$ are as defined in claim 1.

4. A process as claimed in claim 3, characterized in that the palladium catalyst is Pd(0)-triisopropylphosphine.

**Revendications**

1. Procédé pour la préparation de 1,7-octadiène par hydrodimérisation de 1,3-butadiène en présence d'un catalyseur au palladium, d'une phosphine tertiaire, d'acide formique et d'un solvant qui est un dialcoylsulfoxyde et/ou un dialcoylformamide (par "dialcoyl" on veut dire que les atomes de soufre et d'azote dans ces deux derniers groupes de composés sont reliés à deux atomes de carbone différents) et en l'absence d'une base, caractérisé en ce que la phosphine tertiaire est une phosphine de la formule:

$$R_1R_2R_2P$$

dans laquelle $R_1$ est un groupe alcoyle ramifié ayant de 3à 10 atomes de carbone, la ramification se trouvant à un atome de carbone qui n'est pas situé à plus de deux atomes de carbone de l'atome de

phosphore et $R_2$ et $R_3$ sont identiques à $R_1$ ou sont choisis indépendamment parmi des groupes alcoyle, alcényle, aralcoyle, cycloalcoyle ou aryle ayant de 1 à 10 atomes de carbone.

2. Procédé selon la revendication 1, caractérisé en ce que la phosphine tertiaire est la triisopropylphosphine, la di-tert.-butyl-n-butylphosphine, la tri-sec.-butylphosphine ou la tri-tert.-butylphosphine ou un mélange de deux ou plus de ces phosphines.

3. Procédé selon l'une des revendications 1 et 2, caractérisé en ce que le catalyseur au palladium est $Pd(R_1R_2R_3P)_2$ ou $Pd(R_1R_2R_3)_3$, où $R_1$, $R_2$ et $R_3$ sont tels que définis dans la revendication 1.

4. Procédé selon la revendication 3, caractérisé en ce que le catalyseur au palladium est un catalyseur Pd(0)-triisopropylphosphine.

**Patentansprüche**

1. Verfahren zur Herstellung von 1,7-Octadien durch Hydrodimerisierung von 1,3-Butadien in Gegenwart eines Palladium-Katalysators, eines tertiären Phosphins, von Ameisensäure und einem Lösungsmittel, das ein Dialkylsulfoxid und/oder ein Dialkylformamid ist (unter "Dialkyl" ist zu verstehen, dass die Schwefel- bzw. Stickstoffatome der beiden zuletzt genannten Gruppen von Verbindungen mit zwei verschiedenen Kohlenstoffatomen verbunden sind) und in Abwesenheit einer Base, dadurch gekennzeichnet, dass das tertiäre Phosphin ein Phosphin der Formel:

$$R_1R_2R_3P$$

ist, wobei $R_1$ eine verzweigte Alkylgruppe mit 3 bis 10 Kohlenstoffatomen bedeutet, bei der die Verzweigung an einem Kohlenstoffatom vorliegt, das nicht mehr als zwei Kohlenstoffatome von dem Phosphoratom entfernt ist, und $R_2$ und $R_3$ die gleiche Bedeutung besitzen wie $R_1$ oder unabhängig voneinander Alkyl-, Alkenyl-, Aralkyl-, Cycloalkyl- oder Aryl-Gruppen mit 1 bis 10 Kohlenstoffatomen bedeuten.

2. Verfahren nach Anspruch 1, dadurch gekennzeichnet, dass das tertiäre Phosphin Tri-isopropylphosphin, Di-tert.-butyl-n-butylphosphin, Tri-sek.-butylphosphin oder Tri-tert.-butylphosphin oder ein Gemisch aus zwei oder mehreren derartigen Phosphinen ist.

3. Verfahren nach Anspruch 1 oder 2, dadurch gekennzeichnet, dass der Palladium-Katalysator $Pd(R_1R_2R_3P)_2$ oder $Pd(R_1R_2R_3)_3$ ist, wobei $R_1$, $R_2$ und $R_3$ die in Anspruch 1 angegebene Bedeutung haben.

4. Verfahren nach Anspruch 3, dadurch gekennzeichnet, dass der Palladium-Katalysator Pd(0)-Tri-isopropylphosphin ist.